(19) Europäisches Patentamt
European Patent Office
Office européen des brevets

(11) Publication number: 0 051 697 B1

(12) # EUROPEAN PATENT SPECIFICATION

(45) Date of publication of patent specification: 08.02.84

(21) Application number: 80303540.1

(22) Date of filing: 08.10.80

(51) Int. Cl.[3]: C 07 C 120/02, C 07 C 121/407, C 07 C 121/34, C 07 C 121/20, B 01 J 23/02, B 01 J 23/04

(54) ## Hydrocyanation of activated olefins.

(43) Date of publication of application:
19.05.82 Bulletin 82/20

(45) Publication of the grant of the patent:
08.02.84 Bulletin 84/6

(84) Designated Contracting States:
BE CH DE FR GB IT LI LU NL SE

(56) References cited:
US - A - 2 904 581
US - A - 3 278 575
US - A - 3 480 660

HOUBEN-WEYL "Methoden der Organischen Chemie, Sauerstoffverbindungen III" 1952, GEORG THIEME VERLAG, Stuttgart page 271

(73) Proprietor: THE STANDARD OIL COMPANY
Midland Building
Cleveland, Ohio 44115 (US)

(72) Inventor: Pesa, Frederick Anthony
764 Circlewood Drive
Aurora Ohio 44202 (US)
Inventor: Graham, Anne Marie
6290 Greenwood Parkway
103 Northfield Ohio 44067 (US)

(74) Representative: Smith, Sydney et al,
Elkington and Fife High Holborn House 52/54
High Holborn
London WC1V 6SH (GB)

EP 0 051 697 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

# Hydrocyanation of activated olefins

This invention relates to a novel process for the catalytic addition of gaseous hydrogen cyanide to activated olefins.

There are several known methods for hydrocyanating unactivated olefins in the vapor phase. Teter, U.S. Patent 2,385,741, describes the addition of hydrogen cyanide to unactivated olefins in the presence of a finely-divided metallic cobalt catalyst. Darvis, U.S. Patents 3,278,575 and 3,278,576 discloses that finely-divided metallic nickel or palladium can also be employed to catalyze the addition of hydrogen cyanide to unactivated olefins. The highest yield of organic nitriles obtained by any of these processes is 42%.

Furthermore, it is also known in the prior art, i.e. Cordar U.S. Patent 2,904,581, that organic nitriles can be prepared from activated olefins. Activated olefins are hydrocarbons which contain an activating group in close proximity to an olefinic carbon atom. Preferably, this activating group is adjacent to an olefinic carbon. Yields as high as 80% have been obtained by this process. Unfortunately, this is a liquid phase process and both hydrogen cyanide and the activated olefin tend to polymerize at the disclosed reaction conditions. Also, it is very difficult to separate the homogeneous catalyst from the reaction product.

Houben-Weyl, Bond VIII, Methoden Der Organischem Chemie Sauerstoffverbindgungen III, 1952 at pages 266—278 describes the hydrocyanation of various olefinic compounds. Most of the reactions described are liquid phase reactions, some using alkaline catalysts such as KCN. A vapor phase reaction disclosed in Houben-Weyl utilizes an aluminium or silicon dioxide catalyst, preferably activated with a heavy metal salt such as cupric cyanide or zinc cyanide.

The inventive process results in much higher yields of organic nitriles than the prior art vapor phase processes. Furthermore, these higher yields are accomplished at substantially lower temperatures. Finally, since the process of the invention is conducted in the vapor phase the hydrogen cyanide and activated olefin polymerization problem is eliminated.

It has now been found that nitriles can be produced by contacting an activated olefin with gaseous hydrogen cyanide in the presence of a catalyst containing at least one element selected from the group consisting of Group IA and IIA of the Periodic Table.

In a particular embodiment of the invention alpha, beta-unsaturated alkyl acrylates are contacted with gaseous hydrogen cyanide in the presence of a catalyst containing an alkali metal to produce high yields of cyanoesters.

According to the present invention, nitriles are produced by the catalytic addition of hydrogen cyanide to activate olefins. The overall reaction taking place in this process is represented by the following equation:

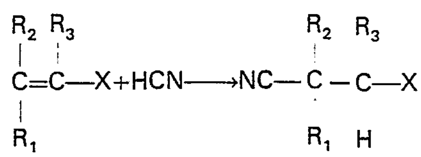

wherein $R_1$, $R_2$, $R_3$ and X are defined below.

Nitriles are well known organic chemicals having a wide variety of uses as solvents and the like. They are also important intermediates for the preparation of a broad spectrum of organic chemicals as described, for example, in the text "The Chemistry of Organic Cyanogen Compounds" by V. Migridichian, American Chemical Society Monograph No. 105, Reinhold Publishing Company (New York, 1947).

Reactants

The preferred activated olefins which can be employed in this invention are compounds of the general formula:

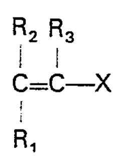

wherein $R_1$, $R_1$ and $R_3$ are each independently selected from the group consisting of:

(1) hydrogen;
(2) $C_{1-4}$ alkyl;
(3) aromatic hydrocarbon radical;
(4) alicyclic hydrocarbon radical;
(5) aralkyl hydrocarbon radical;

(6) 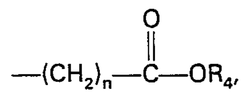

wherein $R_4$ is hydrogen or a $C_{1-4}$ alkyl and n is 0 to 4; and

(7) 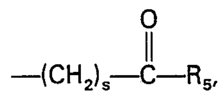

wherein $R_5$ is hydrogen or a $C_{1-4}$ alkyl and s is 0 to 4;
and wherein X is selected from the group consisting of:

(1) 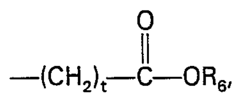

wherein $R_6$ is hydrogen or a $C_{1-12}$ alkyl and t is 0 to 2;

(2) $-(CH_2)_u-CN$, wherein u is 0 or 1; and

(3) 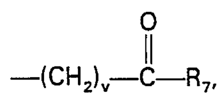

wherein $R_7$ is hydrogen or a $C_{1-12}$ radical and v is 0 to 2.
Examples of these activated olefin include acrolein, methacrolein, acrylonitrile, methacrylonitrile, methyl acrylate and ethyl acrylate.

Preferably, $R_1$, $R_2$ and $R_3$ are each independently selected from:

(1) hydrogen;

(2) $C_{1-4}$ alkyl;

and X is selected from the group consisting of:

(1) 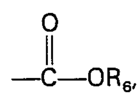

wherein $R_6$ is H or $C_{1-4}$ alkyl;

(2) —CN;

(3) 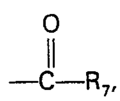

wherein $R_7$ is H or $C_{1-4}$ alkyl.

More preferably, the olefinically unsaturated compounds comprise compounds wherein $R_1$, $R_2$ and $R_3$ are each independently selected from hydrogen and methyl and wherein X is

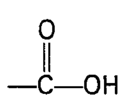 and 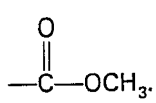

The activated olefin is contacted with gaseous hydrogen cyanide to produce the cyanoester. The ratio of the olefin to hydrogen cyanide is not critical. The instant reaction will proceed with either excess olefin or excess hydrogen cyanide. However, the use of excess activated olefin is preferred, since this minimizes the loss of HCN. Accordingly, it is preferred to employ a molar ratio of activated olefin to HCN of greater than one and preferably between one to ten, in particular from one to two.

If desired, a carrier gas which is inert to the reactants, products and catalysts can be included in the reaction system. Thus, gases such as nitrogen, the noble gases, lower alkanes, carbon monoxide, carbon dioxide, ammonia and minor amounts of hydrogen sulfide can be added to the reaction system.

Process Conditions

In carrying out the inventive process, the activated olefin and hydrogen cyanide in the vapor phase are contacted with a catalyst as described below. This process is preferably conducted on a continuous basis but it can also be accomplished in a batch mode. Either fixed and fluid catalyst beds can be used.

The reaction temperature is normally maintained between 40°C and 350°C, preferably 150°C to

300°C. The reaction pressure is normally maintained at 0 to 7 $kg/cm^2$ (100 psi), preferably 0.7 $kg/cm^2$ to 2.8 $kg/cm^2$ (10 to 40 psi). When the process is carried out in the continuous basis, the contact time is normally ten seconds to ten minutes, preferably 10 seconds to 5 minutes. When the reaction is carried out in a batch mode, reactants and catalysts are contacted with one another for a period of 10 minutes to 6 hours, preferably $\frac{1}{2}$ hour to 4 hours. A reaction time of less than 10 minutes or more than 6 hours can be used if desired, although better results will be obtained if the reaction time is maintained within this range.

Catalyst

The heterogeneous catalyst employed in this process comprises at least one element selected from the group consisting of Group IA to IIA of the Periodic Table or a mixture of elements from each Periodic Table. Preferred catalysts comprise at least one of K, Li, Cs, Ca, Ba and Mg. Especially preferred catalysts contain at least one of K, Li and Cs. This catalyst can be promoted with a wide variety of metals selected from groups VIB, VIIB, VIII and IB. Preferred promoters include Ru, Cr, Mn, Cu, Ni and Ti.

The catalyst used according to the present invention is primarily a compound of a metal capable of withstanding the temperatures employed. These various metal compounds include oxides, hydroxides, and various salts, e.g. ferrocyanides, phophates, carbonates, silicates, aluminates, cyanides and salts of organic acids such as the acetates, formates and butyrates. Preferred catalysts contain oxygen or oxygen-containing anions. The free metals may also be used. Compounds which are somewhat basic in nature or which at least in part react with hydrogen cyanide to form metal cyanides have been found to be particularly good.

The catalyst can be prepared by methods well known in the art. For example, these catalysts can be produced by evaporating a solution of a soluble compound on an inert carrier, or mixing an aqueous slurry of an insoluble compound or of the free metal with an inert carrier, filtering, pressing, drying and calcining the filter cake, and finally grinding the filter cake to the desired particle size. The inert carriers which may be used with this catalyst include aluminates, silicates, titanates and phosphates and mixtures thereof. Preferred carriers are aluminates and silicates. Basic compounds such as quartz, diatomaceous earth, various clays and pumice may also be used.

Recovery

The reaction product obtained upon completion of this reaction are partially in the gas phase. This reaction product can be subjected to suitable known separation techniques to obtain the desired end product.

For example, the product can be condensed to a liquid. The liquid product can then be filtered to remove catalyst therefrom and then separated into component parts by the use of solvent extraction and distillation.

SPECIFIC EMBODIMENTS

In order to more clearly illustrate the present invention, the following working examples are presented. In these examples, the following definition is used:

$$\text{Yield} = \frac{\text{Moles of Nitrile Product Formed}}{\text{Moles of HCN Fed}}$$

Example 1

A catalyst comprising 7.6% LiOAc on a low surface area alumina was prepared as follows. First, 4.11 grams of LiOAc were dissolved in 20 grams of water. Next, 50 grams of a low surface area alumina, i.e. alumina with a surface area of less than 5 square meters per gram, were stirred into the lithium solution. This mixture was dried for 15 hours at 125°C and calcined for 16 hours at 350°C.

40 cc. of the above catalyst were packed into a reactor. A furnace heated the reactor while nitrogen flowed over the catalyst bed. When the reaction temperature reached 250°C, the gas was changed to a mixture of 7% to 10% HCN in nitrogen, and the system was allowed to equilibrate for 15 minutes. Methyl acrylate was then pumped in at a rate of 10 cc. per hour. The output of the reactor was passed to a pair of dry ice cooled glass condensors, and the product was collected. The product was warmed to room temperature, weighed and analyzed. The results are shown in Table 1.

Example 2

A catalyst comprising 11.2 weight percent $KNO_3$ on a low surface area alumina was prepared as follows. First, 6.31 grams of $KNO_3$ were dissolved in 20 grams of water. Next, 50 grams of a low surface area alumina, i.e. alumina with a surface area of less than 5 square meters per gram, were mixed with the aqueous potassium solution. This mixture was dried for 15 hours at 125°C and calcined for 5 hours at 260°C and 15 hours at 538°C.

The catalyst prepared above was placed into the experimental apparatus described in Example 1. The results are shown in Table I.

Examples 3, 4 and 5

Other catalysts containing Group IA or Group IIA elements were prepared by the techniques described in Example 2. These catalysts were also placed in the experimental apparatus described in Example 1 and the results are shown in Table I.

Example 6

A catalyst comprising $MgAl_2O_4$ was prepared as follows. First, 85.47 grams of $Mg(NO_3)_2 \cdot 6H_2O$ were dissolved in water. Next, 33.99 grams of $Al_2O_3$ powder were slurried in water. These two aqueous solutions were mixed together and evaporated to the consistency of toothpaste. The mixture was then dried for 15 hours at 125°C and calcined 5 hours at 260°C (500°F) and 20 hours at 538°C (1,000°F). This catalyst was placed in the experimental apparatus described in Example 1 and the results are shown in Table I.

Examples 7 and 8

Catalysts containing potassium were prepared by the technique described in Example 6. These catalysts were also placed in the experimental apparatus described in Example 1 and the results are shown in Table 1.

Example 9

A catalyst comprising 23.78 weight percent of $KMn(CN)_6$ on a low surface area alumina was prepared as follows. First, 15.6 grams $KMn(CN)_6$ were dissolved in 20 grams of water. 50 grams of a low surface area alumina were mixed with this aqueous solution. The mixture was then dried for 15 hours at 125°C and calcined for 3 hours at 350°C. This catalyst was placed in the experimental apparatus described in Example 1 and the results are shown in Table 1.

Examples 10, 11 and 12

Other catalysts containing potassium were prepared and placed in the experimental apparatus described in Example 1. The results are shown in Table I.

TABLE I

Various Catalysts Useful for the Hydrocyanation of Methyl Acrylate

Methyl Acrylate Feed Rate: 10 cc/hr
HCN Feed Rate: 50 cc/min (7% to 10% HCN, 90% to 93% $N_2$)
Temperature: 250°C.
Pressure: Atmospheric

| Example | Catalyst | Yield (%) |
|---|---|---|
| 1 | 7.60% LiOAc on LSAA[1] | 80.4 |
| 2 | 11.20% $KNO_3$ on LSAA | 98.4 |
| 3 | 19.57% $CsNO_3$ on LSAA | 71.5 |
| 4 | 22.76% $Ca(NO_3)_2$ on HSAA[2] | 60.9 |
| 5 | 24.60% $Ba(NO_3)_2$ on LSAA | 64.7 |
| 6 | $MgAl_2O_4$ | 43.3 |
| 7 | $K_2Si_2O_5$ | 73.2 |
| 8 | $K_2Al_2O_4$ | 53.8 |
| 9 | 23.78% $KMn(CN)_6$ on LSAA | 58.1 |
| 10 | 10.81% $K_2CrO_4$ on HSAA | 90.6 |
| 11 | 12.72% $K_4Ru(CN)_6$ on HSAA | 25.5 |
| 12 | 8.92% $K_2HPO_4$ on LSAA | 75.6 |

[1]LSAA = Low Surface Area Alumina (approximately 5 sq. m/gm.)
[2]HSAA = High Surface Area Alumina (approximately 100 sq. m/gm.)

### Examples 13 to 18

A catalyst comprising $CaAl_2O_4$ was prepared as follows. First, 78.72 grams of $Ca(NO_3)2.4H_2O$ were dissolved in water. Also, 33.99 grams of $Al_2O_3$ powder were slurried in water. These two aqueous solutions were mixed together and evaporated to the consistency of toothpaste. This mixture was then dried for 15 hours at 125°C and calcined for 5 hours at 260°C (500°F) and 20 hours at 538°C (1,000°F). The catalyst was then placed in the experimental apparatus described in Example 1 except that the process conditions were adjusted as shown in Table II. The results of these experiments are shown in Table II.

### Examples 19 to 22

Other catalysts were also tested under various process conditions. These results are shown in Table II.

TABLE II

Various Process Conditions for the Hydrocyanation of Methyl Acrylate (MA)

HCN Feed: 7% to 10% HCN, 90% to 93% $N_2$
Pressure: 1 atmosphere

| Example | Catalyst | Temp. (°C) | Methyl Acrylate Flow Rate (cc/Hr) |
|---|---|---|---|
| 13 | $CaAl_2O_4$ | 175 | 10 |
| 14 | " | 200 | 10 |
| 15 | " | 225 | 10 |
| 16 | " | 250 | 10 |
| 17 | " | 275 | 10 |
| 18 | " | 200 | 10 |
| 19 | 7.60% LiOAc on LSAA | 250 | 10 |
| 20 | " | 250 | 10 |
| 21 | 11.20% $KNO_3$ on LSAA | 250 | 10 |
| 22 | " | 250 | 2.1 |

| Example | HCN Flow Rate (cc/Min) | Yield (%) |
|---|---|---|
| 13 | 50 | 15.2 |
| 14 | 50 | 27.1 |
| 15 | 50 | 46.7 |
| 16 | 50 | 74.8 |
| 17 | 50 | 54.6 |
| 18 | 100 | 13.5 |
| 19 | 50 | 88.8 |
| 20 | 25 | 81.6 |
| 21 | 50 | 79.5 |
| 22 | 50 | 49.2 |

Examples 23 to 25

The catalyst and experimental apparatus shown in Example 2 were used in these examples. Three different activated olefins were tested and each of them yielded some nitrile. The results are shown in table III.

TABLE III

Hydrocyanation of Other Activated Olefins

HCN Feed Rate: 50 cc/min (7% to 10% HCN in nitrogen)
Olefin Feed Rate: 10 ml/hr
Catalyst: 11.20% $KNO_3$ on HSAA
Temperature: 150°C
Pressure: Atmospheric

| Example | Activated Olefin | Yield (%) of Nitrile |
|---|---|---|
| 23 | Acrylic Acid | Approximately 1 |
| 24 | Acrolein | Approximately 1 |
| 25 | Acrylonitrile | Approximately 1 |

**Claims**

1. A process for producing nitriles comprising contacting an activated olefin with gaseous hydrogen cyanide in the vapor phase in the presence of a catalyst containing at least one element selected from the group consisting of Groups IA and IIA of the Periodic Table.

2. A process as claimed in claim 1 characterised in that the activated olefin is represented by the following formula:

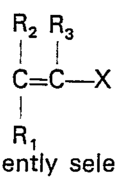

wherein $R_1$, $R_2$ and $R_3$ are each independently selected from:
(1) hydrogen;
(2) $C_{1-4}$ alkyl;
(3) aromatic hydrocarbon radical;
(4) alicyclic hydrocarbon radical;
(5) aralkyl hydrocarbon radical;

(6) 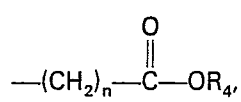

wherein $R_4$ is either H or a $C_{1-4}$ alkyl and n is 0 to 4; and

(7) 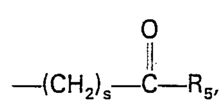

wherein $R_5$ is either H or a $C_{1-4}$ alkyl and s is 0 to 4;
and wherein X is selected from the group consisting of:

(1) 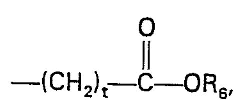

wherein $R_6$ is either H or a $C_{1-12}$ alkyl and t is 0 to 2;
(2) $-(CH_2)_u-CN$, wherein u is 0 to 2; and

(3) 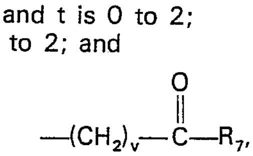

wherein $R_7$ is either H or a $C_{1-12}$ alkyl and v is 0 to 2.

3. A process as claimed in claim 2 characterised in that $R_1$, $R_2$ and $R_3$ independently represent

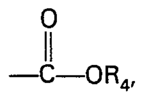

wherein $R_4$ is either H or a $C_{1-4}$ alkyl group.

4. A process as claimed in claim 2 characterised in that $R_1$, $R_2$ and $R_3$ independently represent H or $C_{1-4}$ alkyl.

5. A process as claimed in claim 4 characterised in that $R_1$, $R_2$ and $R_3$ independently represent H or $CH_3$.

6. A process as claimed in any of claims 2 to 5 characterised in that X is selected from the following:

(1) 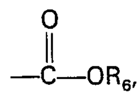

wherein $R_6$ is either H or a $C_{1-4}$ alkyl;

(2) —CN; or

(3) 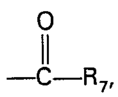

wherein $R_7$ is either H or a $C_{1-4}$ alkyl

7. A process as claimed in claim 6 characterised in that X is

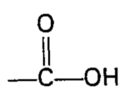 or 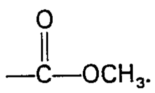

8. A process as claimed in any of claims 1 to 7 characterised in that the molar ratio of activated olefin to hydrogen cyanide is between 1:1 and 2:1.

9. A process as claimed in any of claims 1 to 8 characterised in that the catalyst is heterogeneous.

10. A process as claimed in any of claims 1 to 9 characterised in that the reaction is carried out at a temperature between 150°C and 300°C.

11. A process as claimed in any of claims 1 to 10 characterised in that the reaction is carried out at a pressure of from 0.7 kg/cm$^2$ (10 psi) and 2.8 kg/cm$^2$ (40 psi).

12. A process as claimed in any of claims 1 to 11 characterised in that the catalyst comprises a mixture of Group IA and Group IIA elements.

13. A process as claimed in any of claims 1 to 12 characterised in that the catalyst comprises at least one of the following metals: Li, Na, K, Rb, Cs, Mg, Ca, Sr and Ba.

14. A process as claimed in any of claims 1 to 13 characterised in that the catalyst is supported on an inert carrier.

15. A process as claimed in claim 14 characterised in that the carrier is alumina, silica or alumina silicate.

16. A process as claimed in any of claims 1 to 15 characterised in that a cyanoester is produced.

17. A process for producing a cyanoester comprising contacting an alpha, beta-unsaturated alkyl acrylate with gaseous hydrogen cyanide in the presence of a catalyst containing an alkali metal component.

**Revendications**

1. Procédé pour préparer des nitriles, caractérisé par le fait qu'il consiste à mettre en contact une oléfine activée avec du gaz cyanhydrique en phase vapeur en présence d'un catalyseur contenant au moins unélément choisi parmi les éléments appartenant au groupe IA et au groupe IIA de la Classification Périodique.

2. Procédé selon la revendication 1, caractérisé par le fait que l'oléfine activée est représentée par la formule suivante:

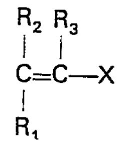

dans laquelle $R_1$, $R_2$ et $R_3$ sont chacun indépendamment choisis parmi:

(1) hydrogène;
(2) alkyle en $C_{1-4}$;
(3) radical hydrocarboné aromatique;
(4) radical hydrocarboné alicyclique;
(5) radical hydrocarboné arylalkyle;

(6) 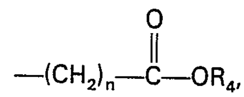

dans lequel $R_4$ est un hydrogène ou un alkyle en $C_{1-4}$ et n est 0 à 4; et

(7) 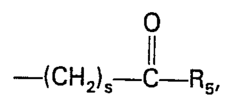

dans lequel $R_5$ est un hydrogène ou un alkyle en $C_{1-4}$ et s est 0 à 4; et dans laquelle X est choisi dans le groupe comprenant:

(1) 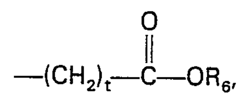

dans lequel $R_6$ est un hydrogène ou un alkyle en $C_{1-12}$ et t est 0 à 2;

(2) $-(CH_2)_u-CN$, dans lequel u est 0 à 2; et

(3) 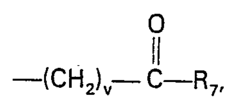

dans lequel $R_7$ est un hydrogène ou un radical en $C_{1-12}$ et v est 0 à 2.

3. Procédé selon la revendication 2, caractérisé par le fait que $R_1$, $R_2$ et $R_3$ représentent indépendamment

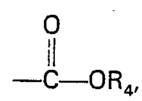

dans laquelle $R_4$ est H ou un groupe alkyle en $C_{1-4}$.

4. Procédé selon la revendication 2, caractérisé par le fait que $R_1$, $R_2$ et $R_3$ représentent indépendamment H ou un alkyle en $C_{1-4}$.

5. Procédé selon la revendication 4, caractérisé par le fait que $R_1$, $R_2$ et $R_3$ représentent indépendamment H ou $CH_3$.

6. Procédé selon l'une des revendications 2 à 5, caractérisé par le fait que X est choisi parmi les groupes suivants:

(1) 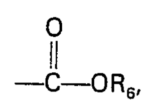

dans lequel $R_6$ est H ou alkyle en $C_{1-4}$;

(2) $-CN$;

(3) 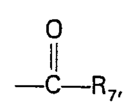

dans lequel $R_7$ est H ou alkyle en $C_{1-4}$.

7. Procédé selon la revendication 6, caractérisé par le fait que X est:

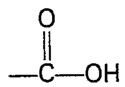 ou 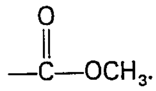

8. Procédé selon l'une des revendications 1 à 7, caractérisé par le fait que le rapport molaire entre l'oléfine activée et le gaz cyanhydrique est compris entre 1:1 et 2:1.

9. Procédé selon l'une des revendications 1 à 8, caractérisé par le fait que le catalyseur est hétérogène.

10. Procédé selon l'une des revendications 1 à 9, caractérisé par le fait que l'on effectue la réaction à une température entre 150°C et 300°C.

11. Procédé selon l'une des revendications 1 à 10, caractérisé par le fait que l'on effectue la réaction sous une pression comprise entre 0,69 bar (0,7 kg/cm²) et 2,76 bars (2,8 kg/cm²).

12. Procédé selon l'une des revendications 1 à 11, caractérisé par le fait que le catalyseur comprend un mélange d'éléments du groupe IA et du groupe IIA.

13. Procédé selon l'une des revendications 1 à 12, caractérisé par le fait que le catalyseur comprend au moins l'un des métaux suivants: Li, Na, K, Rb, Cs, Mg, Ca, Sr et Ba.

14. Procédé selon l'une des revendications 1 à 13, caractérisé par le fait que le catalyseur est supporté sur un véhicule inerte.

15. Procédé selon la revendication 14, caractérisé par le fait que le véhicule est de l'alumine, de la silice ou du silicate d'aluminium.

16. Procédé selon l'une des revendications 1 à 15, caractérisé par le fait qu'on prépare un cyanoester.

17. Procédé pour préparer un cyanoester, caractérisé par le fait qu'il consiste à mettre en contact un acrylate d'alkyle alpha, bêta-insaturé avec du gaz cyanhydrique en présence d'un catalyseur contenant comme composant un métal alcalin.

## Patentansprüche

1. Verfahren zur Herstellung von Nitrlien, bei dem ein aktiviertes Olefin mit gasförmigem Cyanwasserstoff in der Dampfphase in Gegenwart eines Katalysators in Kontakt gebracht wird, der mindestens ein Element enthält, das ausgewählt wird aus der Gruppe, die besteht aus den Gruppen IA und IIA des Periodischen Systems der Elemente.

2. Verfahren nach Anspruch 1, dadurch gekennzeichnet, daß das aktivierte Olefin dargestellt wird durch die folgende Formel

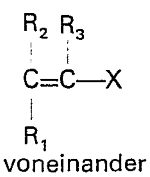

worin $R_1$, $R_2$ und $R_3$ jeweils unabhängig voneinander ausgewählt werden aus:

(1) Wasserstoff;
(2) $C_{1-4}$ Alkyl;
(3) aromatischer Kohlenwasserstoffrest;
(4) alicyclischer Kohlenwasserstoffrest;
(5) Aralkylkohlenwasserstoffrest;

(6) 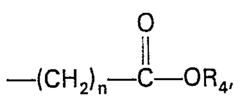

worin $R_4$ entweder H oder ein $C_{1-4}$-Alkyl und n 0 bis 4 darstellen; und

(7) 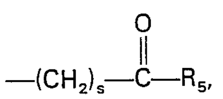

worin $R_5$ entweder H oder ein $C_{1-4}$-Alkyl und s 0 bis 4 darstellen;
und worin X ausgewählt wird aus der Gruppe, die besteht aus:

(1) 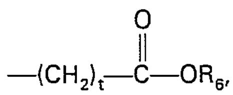

worin $R_6$ H oder ein $C_{1-12}$-Alkyl und t 0 bis 2 darstellen;

(2) $-(CH_2)_u-CN$, worin u 0 bis 2 darstellt; und

(3) 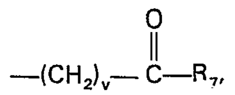

worin $R_7$ H oder ein $C_{1-12}$-Alkyl und v 0 bis 2 darstellen.

3. Verfahren nach Anspruch 2, dadurch gekennzeichnet, daß $R_1$, $R_2$ und $R_3$ unabhängig voneinander

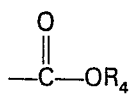

bedeuten, worin $R_4$ H oder eine $C_{1-4}$-Alkylgruppe darstellen.

4. Verfahren nach Anspruch 2, dadurch gekennzeichnet, daß $R_1$, $R_2$ und $R_3$ unabhängig voneinander H oder $C_{1-4}$-Alkyl bedeuten.

5. Verfahren nach Anspruch 4, dadurch gekennzeichnet, daß $R_1$, $R_2$ und $R_3$ unabhängig voneinander H oder $CH_3$ bedeuten.

6. Verfahren nach einem der Ansprüche 2 bis 5, dadurch gekennzeichnet, daß X ausgewählt wird aus:

(1) 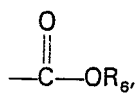

worin $R_6$ H oder ein $C_{1-4}$-Alkyl darstellt;

(2) $-CN$; oder

(3) 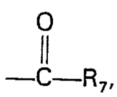

worin $R_7$ H oder ein $C_{1-4}$-Alkyl darstellt.

7. Verfahren nach Anspruch 6, dadurch gekennzeichnet, daß X

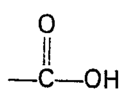 oder 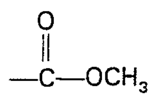

bedeutet.

8. Verfahren nach einem der Ansprüche 1 bis 7, dadurch gekennzeichnet, daß das Molverhältnis von aktiviertem Olefin zu Cyanwasserstoff zwischen 1:1 und 2:1 liegt.

9. Verfahren nach einem der Ansprüche 1 bis 8, dadurch gekennzeichnet, daß der Katalysator heterogen ist.

10. Verfahren nach einem der Ansprüche 1 bis 9, dadurch gekennzeichnet, daß die Reaktion bei einer Temperatur zwischen 150°C und 300°C durchgeführt wird.

11. Verfahren nach einem der Ansprüche 1 bis 10, dadurch gekennzeichnet, daß die Reaktion bei einem Druck von 0,7 kg/cm² (10 psi) und 2,8 kg/cm² (40 psi) durchgeführt wird.

12. Verfahren nach einem der Ansprüche 1 bis 11, dadurch gekennzeichnet, daß der Katalysator eine Mischung von Elementen der Gruppe IA und der Gruppe IIA umfaßt.

13. Verfahren nach einem der Ansprüche 1 bis 12, dadurch gekennzeichnet, daß der Katalysator mindestens eines der folgenden Metalle enthält: Li, Na, K, Rb, Cs, Mg, Ca, Sr und Ba.

14. Verfahren nach einem der Ansprüche 1 bis 13, dadurch gekennzeichnet, daß der Katalysator auf einen inerten Träger aufgebracht ist.

15. Verfahren nach Anspruch 14, dadurch gekennzeichnet, daß der Träger Aluminiumoxid, Siliziumdioxid oder Aluminasilikat ist.

16. Verfahren nach einem der Ansprüche 1 bis 15, dadurch gekennzeichnet, daß ein Cyanoester hergestellt wird.

17. Verfahren zur Herstellung eines Cyanoesters, bei dem man ein Alpha, Beta-ungesättigtes Alkylacrylat mit gasförmigem Cyanwasserstoff in Gegenwart eines Katalysators in Kontakt bringt, der eine Alkalimetallkomponente enthält.